# EUROPEAN PATENT APPLICATION

(11) **EP 3 884 911 A1**
(43) Date of publication of application: **29.09.2021**
(21) Application number: 20165766.5
(22) Date of filing: 26.03.2020
(51) Int. Cl.: A61F 2/95

(54) **STENT DELIVERY SYSTEM AND HANDLING DEVICE FOR A STENT DELIVERY SYSTEM**

(71) Applicant: B. Braun Melsungen AG, 34212 Melsungen (DE)
(72) Inventor: Murphy, Brian, Bray, Co. Wicklow, A 98 Y6WO (IE)
(74) Representative: Patentanwälte Ruff, Wilhelm, Beier, Dauster & Partner mbB

(57) **Abstract**

Stent delivery system, comprising: a handling device having a housing, a thumbwheel mounted in the housing to be rotatable, and a winding spool rotating together with the thumbwheel; a catheter arrangement having an inner shaft, with a proximal end of the inner shaft fixed on the housing, an outer sheath disposed coaxially to the inner shaft, and having at least one stent which is received radially between the inner shaft and the outer sheath; a flexible pull member which, with one end, engages on a proximal end of the outer sheath and, with the other end, is held on the winding spool to be windable; wherein, for release of at least the one stent, the outer sheath is displaceable relative to the inner shaft in the proximal direction by means of winding the flexible pull member on the winding spool; wherein a deflecting and tensioning device is provided spaced from the winding spool in the proximal direction, which deflecting and tensioning device is in operative connection to the flexible pull member, and is used to deflect the flexible pull member in a direction towards the winding spool and to tension the flexible pull member.

## Description

### TECHNICAL FIELD AND PRIOR ART

The invention relates to a stent delivery system and to a handling device for a stent delivery system.

It is well known to employ intravascular endoprostheses delivered percutaneously for the treatment of diseases of various body vessels. Such intravascular endoprostheses are commonly referred to as "stents". A stent is a generally longitudinal tubular device of biocompatible material having holes or slots that define a flexible framework that allows radial expansion of the stent, by a balloon catheter or the like, or alternatively by self-expansion due to shape memory characteristics of the material within the body vessel. The flexible framework is configured to allow the stent to be compressed into a smaller outer diameter so that it can be mounted inside a stent delivery system.

The stent delivery system is used to convey the stent to a desired location within the body vessel, and then to release the stent in position. Upon release the stent may self-expand into a larger outer diameter.

WO 2019/053508 A1 discloses a stent delivery system having a handling device and a catheter arrangement. The catheter arrangement comprises an inner shaft, an outer sheath disposed coaxially to the inner shaft, and a stent which is received radially between the inner shaft and the outer sheath. The inner shaft of the catheter arrangement has a proximal end that is fixed to a housing of the handling device. The stent delivery system further comprises a flexible pull member which, with one end, engages on a proximal end of the outer sheath and, with the other end, is held on a winding spool of the handling device. The winding spool is operatively connected to a thumbwheel that is rotatably mounted inside the housing of the handling device. For release of the stent, the thumbwheel is rotated manually, whereby the winding spool is rotated. The flexible pull member engaging on the winding spool is thereby wound onto the winding spool and, as a result, the outer sheath is displaced relative to the inner shaft in the proximal direction. Due to this proximal retraction of the outer sheath, the stent is released and thus can expand within the blood vessel.

### SUMMARY OF THE INVENTION

It is the object of the present invention to provide a stent delivery system that allows a secure handling during the release of the stent. It is a further object of the present invention to provide a handling device for such a stent delivery system, the handling device allowing a secure handling during the release of the stent.

According to a first aspect, a stent delivery system is provided comprising: a handling device having a housing, a thumbwheel mounted in the housing to be rotatable, and a winding spool rotating together with the thumbwheel; a catheter arrangement having an inner shaft, with a proximal end of the inner shaft fixed on the housing, an outer sheath disposed coaxially to the inner shaft, and having at least one stent which is received radially between the inner shaft and the outer sheath; a flexible pull member which, with one end, engages on a proximal end of the outer sheath and, with the other end, is held on the winding spool to be windable; wherein, for release of at least the one stent, the outer sheath is displaceable relative to the inner shaft in the proximal direction by means of winding the flexible pull member on the winding spool; wherein a deflecting and tensioning device is provided spaced from the winding spool in the proximal direction, which deflecting and tensioning device is in operative connection to the flexible pull member, and is used to deflect the flexible pull member in a direction towards the winding spool and to tension the flexible pull member. The solution according to the invention ensures that the proximal end of the outer shaft during winding of the flexible pull member in the proximal direction can be displaced beyond the position of the winding spool. For that purpose, the deflecting and tensioning device is spaced from the winding spool in the proximal direction, wherein the flexible pull member is deflected, starting from the proximal end of the outer sheath and a proximal extension in the direction towards the winding spool. At the same time, the solution according to the invention ensures pre-tensioning of the flexible pull member. For that purpose, the flexible pull member is pre-tensioned using the deflecting and tensioning device. Thereby, an actuation of the thumbwheel free of play is allowed and unintended coming off of the flexible pull member from the winding spool is prevented. As a result, the solution according to the invention allows an exact release of the stent and finally secure handling. The deflecting and tensioning device is intended on the one hand for deflecting and on the other hand for pre-tensioning of the flexible pull member and, thus, presents a particularly advantageous multifunction. Thereby, specific devices for deflecting and for tensioning of the flexible pull member can be omitted and a simple structural design can be achieved.

In one embodiment, the deflecting and tensioning device is disposed completely inside the housing. Thereby, the deflecting and tensioning device is manually not accessible, in any case in a ready-for-use condition of the stent delivery system. An inadvertent manipulation of the deflecting and tensioning device and, in particular a variation of the pre-tensioning of the flexible pull member accompanied therewith, is prevented thereby.

In one embodiment, the deflecting and tensioning device includes a base body fixed in the housing and a tensioning element acting on the flexible pull member, which tensioning element is mounted on the base body to be movable relative to the base body between different tensioning positions, wherein the flexible pull member in the different tensioning positions is tensioned by means of the tensioning element to a different extent. The base body can be fixed to the housing in a force-fitting, form-fitting and/or integrally bonded manner. The relative movement of the tensioning element in relation to the base body can be a linear, rotational and/or pivoting movement. The tensioning element can be fixed in the different tensioning positions relative to the base body. In the different tensioning positions the tensioning element acts on the flexible pull member to different extent such that the latter is pre-tensioned to a different extent. For pre-tensioning of the flexible pull member, the tensioning element is displaced relative to the base body and fixed thereon, once the required pre-tensioning is achieved. This occurs preferably manually and preferably on the manufacturing side, i.e. during manufacturing and/or assembly of the stent delivery system. That is, preferably an adjustment of the pre-tensioning and, thus, an actuation of the deflecting and tensioning device by the user of the stent delivery system is not intended.

In one embodiment, the tensioning element is mounted on the base body to be pivotable about a pivoting axis. Thereby, a particularly simple construction of the deflecting and tensioning device is possible. In particular in case that a radial action of the tensioning element on the flexible pull member is provided, a lever action can be achieved by pivotable mounting of the tensioning element on the base body. This is advantageous in view of an achievable pre-tensioning of the flexible pull member.

In one embodiment, the tensioning element includes a tensioning section which acts radially on the flexible pull member, whereby the flexible pull member is tensioned. In the different tensioning positions the tensioning section acts radially on the flexible pull member to a different extent. The flexible pull member is thereby deployed laterally - with reference to a straight extension between the deflecting and tensioning device and the winding spool - whereby a pre-tensioning of different intensity is obtained. In the axial direction, the flexible pull member preferably cooperates with the tensioning section in sliding movement.

In one embodiment, the tensioning element includes a deflecting section on which the flexible pull member is deflected in sliding from an extension in the proximal direction towards the direction of the winding spool. The flexible pull member extends starting from the proximal end of the outer sheath in the direction of the deflecting section, rests in sliding on the latter and is deflected in the direction of the winding spool. Preferably, the flexible pull member is deflected by approximately 180° on the deflecting section. In order to prevent excessive wear of the flexible pull member, the deflecting section preferably has a circular cylindrical outer contour on which the flexible pull member is guided along in sliding.

In one embodiment, the base body includes a passage extending between a distal end and a proximal end of the base body, wherein the inner shaft and the flexible pull member extend through the passage. This embodiment of the invention allows arrangement of the deflecting and tensioning device with particularly low space occupation.

In one embodiment, the deflecting and tensioning device includes a ratchet mechanism provided between the base body and the tensioning element, which ratchet mechanism allows movement of the tensioning element between the different tensioning positions in a first direction to increase the tension of the flexible pull member, and counteracts movement of the tensioning element in an opposite second direction. This is a particularly preferred embodiment of the invention. The ratchet mechanism is preferably disposed completely inside the housing. As a result, an inadvertent manipulation of the ratchet mechanism is prevented. Preferably, the ratchet mechanism is operable exclusively with the housing in an opened condition. For example, this is during manufacture and/or assembly of the stent delivery system. Since the ratchet mechanism counteracts movement of the tensioning element in the second direction, separate fastening of the tensioning element in the tensioning position required for pre-tensioning of the flexible pull member can be omitted.

In one embodiment, the ratchet mechanism includes a first ratchet surface provided on the base body and a second ratchet surface provided on the tensioning element, which second ratchet surface cooperates to register with the first ratchet surface along the first direction, and which is locked in a form-fitting manner against the first ratchet surface along the second direction. This embodiment of the invention allows a particularly simple and robust configuration of the ratchet mechanism and, thus, of the deflecting and tensioning device. The first ratchet surface and the second ratchet surface are preferably each provided with a toothing. These toothing features are designed complementary to each other and register along the first direction and lock in a form-fitting manner along the second direction.

According to a second aspect, a handling device is provided, the handling device having a housing, a thumbwheel mounted in the housing to be rotatable, and a winding spool rotating together with the thumbwheel, wherein a deflecting and tensioning device is provided spaced from the winding spool in a proximal direction, which deflecting and tensioning device is configured for operative connection to a flexible pull member of a catheter arrangement of the stent delivery system. In order to avoid repetition, reference is made to the description of the first aspect and its embodiments. The explanations given there with respect to the handling device of the stent delivery system apply correspondingly to the handling device according to the second aspect.

### BRIEF DESCRIPTION OF THE DRAWINGS

In the following, an embodiment of the invention will be described in detail with reference to the drawings. Throughout the drawings, the same elements will be denoted by the same reference numerals. The drawings schematically show:
- Fig. 1: in an isometric view an embodiment of a stent delivery system according to the invention, including an embodiment of a handling device according to the invention and a catheter arrangement with a plurality of stents, wherein the catheter arrangement is illustrated with partial sections;
- Fig. 2: the stent delivery system according to Fig. 1 in the region of the handling device in a further isometric view;
- Fig. 3: the stent delivery system according to Figs. 1 and 2 in a view corresponding to Fig. 2 with individual components and/or sections of the handling device hidden in the drawing;
- Fig. 4: a further isometric view similar to Fig. 3 with additional components and/or sections hidden in the drawing and with a direction of view on a flexible pull member and a deflecting and tensioning device associated with the pull member;
- Fig. 5: an enlarged detailed illustration in the region of the deflecting and tensioning device which has a base body and a tensioning element illustrated partially transparent;
- Fig. 6: the base body of the deflecting and tensioning device in an isometric view;
- Fig. 7: the base body according to Fig. 6 in a top view;
- Fig. 8, 9: the deflecting and tensioning device, wherein the tensioning element occupies a first tensioning position (Fig. 8) and a second tensioning position (Fig. 9); and
- Fig. 10: an enlarged detailed illustration of the tensioning element in a bottom view.

### DETAILED DESCRIPTION OF EMBODIMENTS OF THE INVENTION

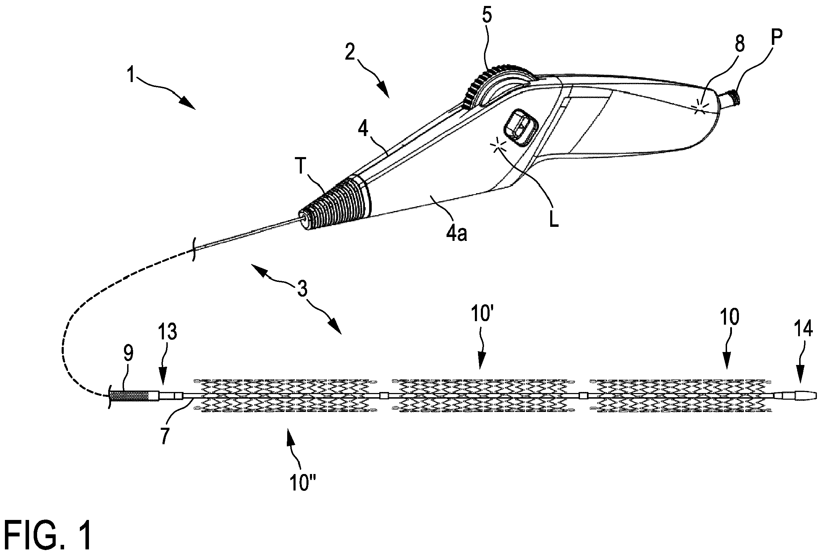

According to Fig. 1, a stent delivery system 1 comprises a handling device 2 and a catheter arrangement 3. The stent delivery system 1 is intended for use in the treatment of stenosis in blood vessels.

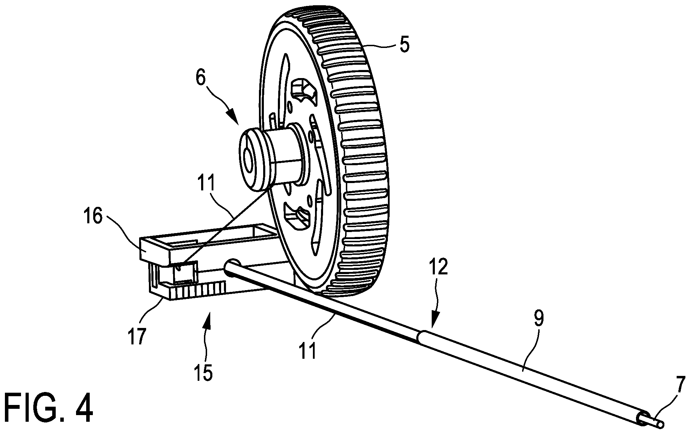

The handling device 2 has a housing 4 with a thumbwheel 5 mounted in the housing 4 to be rotatable and a winding spool 6 rotating together with the thumbwheel 5. The catheter arrangement 3 has an inner shaft 7 with a proximal end 8 which is at least indirectly fixed on the housing 4 in a generally well-known manner. Further, the catheter arrangement 3 has an outer sheath 9 disposed coaxially to the inner shaft 7. The catheter arrangement 3 includes at least one stent 10 which is received radially between the inner shaft 7 and the outer sheath 9 in a condition of the catheter arrangement 3 not graphically illustrated in more detail. Moreover, the stent delivery system 1 includes a flexible pull member 11 (Fig. 4) which, with one end, engages on a proximal end 12 of the outer sheath 9 and, with the other end, is held on the winding spool 6 to be windable. For release of at least the one stent 10, the outer sheath 9 is displaceable relative to the inner shaft 7 in the proximal direction by means of winding the flexible pull member 11 on the winding spool 6. The above-mentioned release takes place starting from a displacement position of the outer sheath 9 not illustrated in more detail, wherein a distal end 13 of the outer sheath 9 is essentially flush with a distal end 14 of the inner shaft 7. In this condition, the stent 10 is compressed in the radial direction between an outer shell surface of the inner shaft 7 and an inner shell surface of the outer sheath 9. Following the above-mentioned retraction of the outer sheath 9 in the proximal direction, the stent 10 is released in the radial direction, and thereby may expand in the radial direction. With reference to Fig. 1, a corresponding released and expanded condition of the first stent 10 is illustrated.

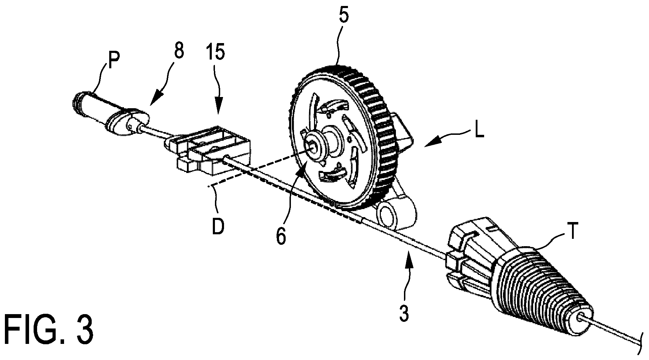

As is in particular illustrated with reference to Figs. 3 and 4, the stent delivery system 1 comprises a deflecting and tensioning device 15 which is disposed spaced from the winding spool 6 in a proximal direction and in operative connection to the flexible pull member 11. The flexible pull member 11 is tensioned by means of the deflecting and tensioning device 15 and deflected from an extension in the proximal direction towards the direction of the winding spool 6.

The pre-tensioning of the flexible pull member 11 caused by the deflecting and tensioning device 15 allows a play-free actuation of the thumbwheel 5 and, thus, a precise control of releasing of the stent 10. Due to the arrangement of the deflecting and tensioning device 15 spaced in the proximal direction and the deflection of the flexible pull member 11 resulting therefrom, the proximal sheath 9 can be displaced further in the proximal direction than would be allowed during a non-deflected winding of the flexible pull member 11 on the winding spool 6.

Before discussing the specific configuration of the handling device 2 and the catheter arrangement 3, the further features of the deflecting and tensioning device 15 are explained.

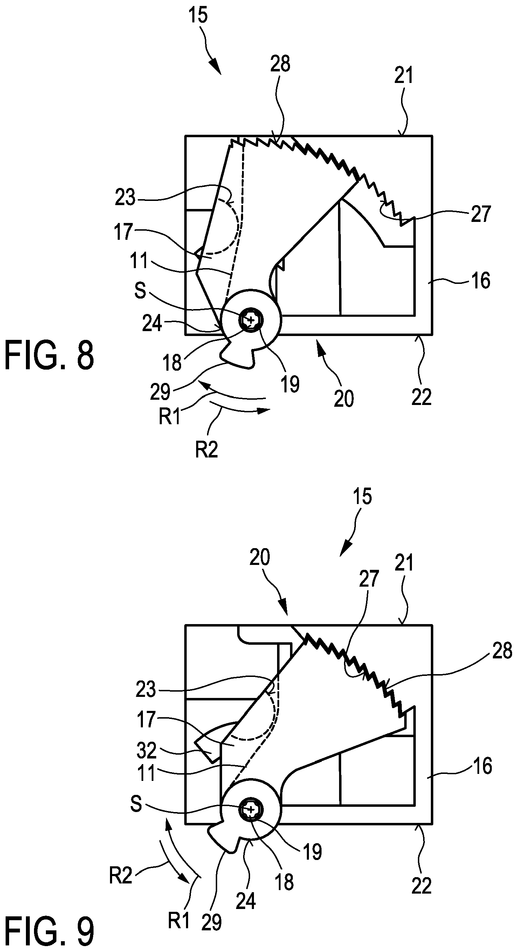

The deflecting and tensioning device 15 includes a base body 16 and a tensioning element 17. The base body 16 is fixed in the housing 4 in a manner described in more detail below. The tensioning element 17 is mounted on the base body 16 and movable relative to the latter between different tensioning positions (Fig. 8, 9). In that context, the tensioning element 17 acts on the flexible pull member 11 to a different extent in the different tensioning positions such that the flexible pull member 11 is accordingly tensioned to a different extent.

In the embodiment shown, the tensioning element 17 is fixed on the base body 16 to be pivotable about a pivoting axis S. The pivoting axis S is provided by a bearing journal 18 disposed on the base body 16, which bearing journal 18 cooperates in sliding with a bearing bore 19 formed on the tensioning element 17.

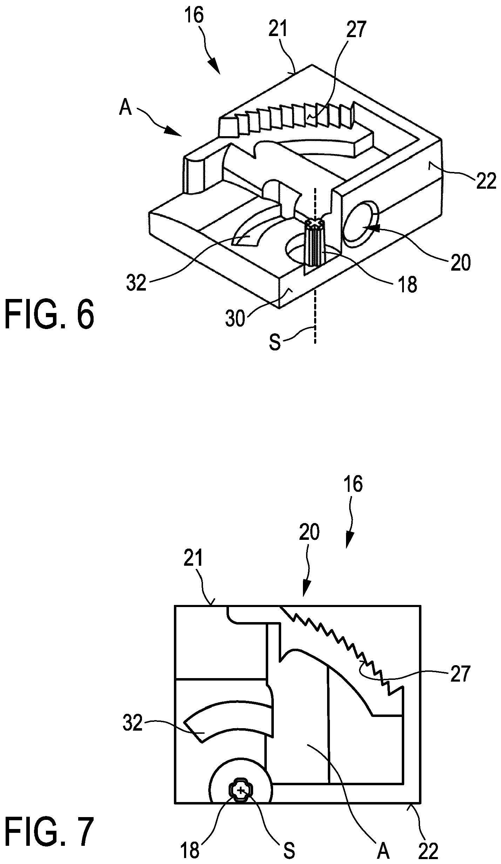

The base body 16 has a cuboid shape (Figs. 6, 7). Further, the base body 16 has a receiving recess A which is disposed on an upper flat face of the cuboid shape, with reference to the drawing plane of Fig. 6. The receiving recess A is provided for receiving the tensioning element 17. The tensioning element 17, in a condition mounted on the base body 16, in which condition the bearing journal 18 engages in the bearing bore 19, is inserted in the receiving recess A flush-mount with the upper flat face of the cuboid shape. This feature is also apparent with reference to Figs. 3 and 4. In addition this illustration shows that the receiving recess A in the ready-for-use assembled condition is oriented downwards. However, this feature is not mandatory.

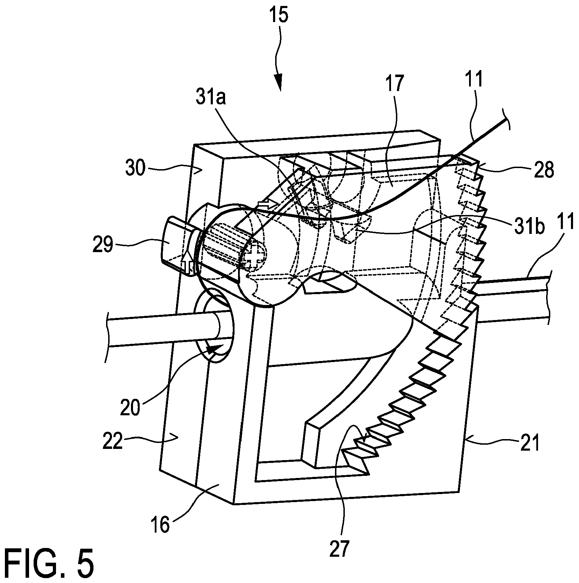

Further, the base body includes a passage 20 extending between a distal end 21 and a proximal end 22 of the base body 16. The passage 20 has a circular cylindrical design in the embodiment shown. Further, the passage 20 is oriented coaxially to the longitudinal direction of extension of the catheter arrangement 3 in the region of the handling device 2. In that context, at least the inner shaft 7 and the flexible pull member 11 extend through the passage 20 (Figs. 4, 5).

In the embodiment shown, the base body 16 manufactured from a dimensionally stable synthetic material. As an alternative, manufacture from metal can be provided.

In the embodiment shown, the tensioning element 17 includes a tensioning section 23 and a deflecting section 24.

The deflecting section 24 is disposed on a proximal end 25 of the tensioning element 17 which in the ready-for-use assembled condition is oriented approximately flush with the proximal end 22 of the base body 16.

Therein, the deflecting section 24 adjoins a proximal end of the passage 20 not described in more detail. For deflection, the flexible pull member 11 is led out of the proximal end of the passage 20 and placed on the deflecting section 24. During rotational actuation of the thumbwheel 5 and winding of the flexible pull member 11 on the winding spool 6 accompanied therewith, the flexible pull member 11 slides along the deflecting section 24. In the embodiment shown, the flexible pull member 11 is deflected by approximately 180° by means of the deflecting section 24.

In the present case, the deflecting section 24 is configured in the shape of a circular cylindrical section which is oriented coaxially to the bearing bore 19.

For pre-tensioning, the tensioning section 23 acts in the radial direction of the flexible pull member 11. This action has a varying extent in the different tensioning positions (Figs. 8, 9). With reference to Figs. 8 and 9, the tensioning section 23 disposed on the bottom side of the tensioning element 17 is concealed by the upper side of the tensioning element 17 and accordingly drawn in dashed lines. The same applies to the flexible pull member 11 which for better illustration is drawn in the region of the deflecting section 24 and the tensioning section 23 likewise in dashed lines and partially sectioned.

In the embodiment shown, the tensioning section 23 is disposed approximately in a central position between the proximal end 25 and a distal end 26 of the tensioning element 17. Further, the tensioning section 23 has a circular cylindrical outer contour corresponding to the deflecting section 24, which outer contour is provided for acting on the flexible pull member 11. During winding of the flexible pull member 11, the latter slides along the tensioning section 23 and along the circular cylindrical outer contour, respectively.

Additionally, the deflecting and tensioning device 15 includes a ratchet mechanism 27, 28 provided between the base body 16 and the tensioning element 17. The ratchet mechanism 27, 28 permits relative movement of the tensioning element 17 in a first direction R1 and counteracts an opposite relative movement in a second direction R2. The directions of movement R1, R2 of the tensioning element 17 are pivoting movements relative to the base body 16 about the pivoting axis S, in the present case. A displacement of the tensioning element 17 along the first direction R1 causes pre-tensioning of the flexible pull member 11.

In the embodiment shown, the ratchet mechanism 27, 28 includes a first ratchet surface 27 provided on the base body 16 and a second ratchet surface 28 provided on the tensioning element 17. The first ratchet surface 27 and the second ratchet surface 28 register along the first direction R1. Along the second direction R2 the ratchet surfaces 27, 28 cooperate in a form-fitting manner such that a movement of the tensioning element 17 in the second direction R2 is blocked.

The second ratchet surface 28 is disposed on the distal end 26 of the tensioning element 17. The first ratchet surface 27 is disposed in the region of the distal end 21 of the base body 16 and faces the proximal direction of the receiving recess A.

The ratchet surfaces 27, 28 have complementary toothing features.

Further, the tensioning element 17 has an abutment section 29 which cooperates in a form-fitting manner with a counter-abutment section 30 of the base body 16 for limiting the pivoting displacement of the tensioning element 17. This feature is illustrated with reference to Fig. 9. The tensioning position of the tensioning element 17 apparent therein is in that context an end position along the first direction R1. In the present case, the abutment section 29 is disposed on the proximal end 25 of the tensioning element 17 and protrudes from the deflecting section 24 outwards in the radial direction. The counter-abutment section 30 is disposed on the proximal end 22 of the base body 16.

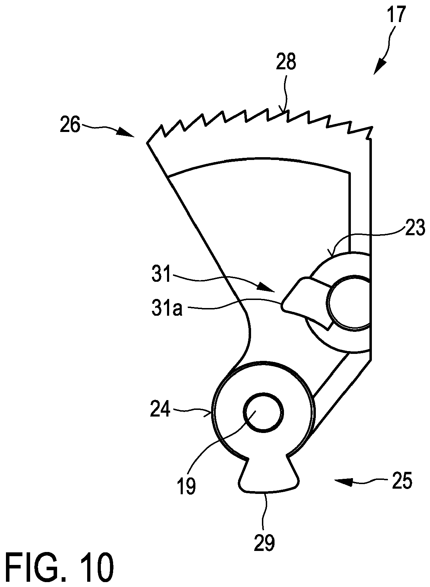

Moreover, the tensioning element 17 includes a guiding section 31 which is disposed in the region of the tensioning section 23 and provided by two guiding partial sections 31a, 31b mutually spaced in the axial direction of the tensioning section 23. By means of the guiding section 31, on the one hand, the flexible pull member 11 is guided in the vicinity of the tensioning section 23 such that an unintended lateral slipping of the flexible pull member 11 is prevented. Moreover, the tensioning element 17 is guided in sliding movement on the base body 16 by means of the guiding section 31 which, for that purpose is provided with a guiding groove 32. The guiding partial section 31a (Fig. 10) engages in sliding in the guiding groove 32.

As is further illustrated in particular with reference to Figs. 3 and 4, in the present case, the deflecting and tensioning device 15 is disposed completely inside the housing 4. In the ready-for-use assembled condition of the housing 4, the deflecting and tensioning device 15 is is manually not accessible by a user of the stent delivery system 1. In that context, manual adjustment of the pre-tensioning of the flexible pull member 11 by a user is also not provided. Rather, adjustment of the pre-tensioning takes place during manufacture and/or assembly of the stent delivery system 1 with the housing 4 in an opened condition. In this opened condition of the housing 4, which in the present case has a left housing half 4a and a right housing half 4b, the deflecting and tensioning device 15 is manually accessible.

Starting from the condition apparent with reference to Fig. 8, wherein the tensioning element 17 occupies a first tensioning position, the tensioning element 17 is displaced by pivoting along the first direction R1 to increase the pre-tensioning. For that purpose, the tensioning element 17 may be moved in the first direction R1, for example, using the thumb of one hand in the region of the distal end 26. What is caused thereby is that the tensioning section 23 acts in the radial direction on the flexible pull member 11 so that the latter - in relation to the drawing plane of Figs. 8 and 9 - is pushed laterally to the right. Thereby, any play in the flexible pull member 11 can be removed and a pre-tensioning be caused. During such a movement of the tensioning element 17, the ratchet surfaces 27, 28 cooperate to register and prevent an opposite movement along the second direction R2.

Further details of the configuration of the handling device 2 and the catheter arrangement 3 will be discussed below, however are not to be considered as mandatory features in view of the present invention.

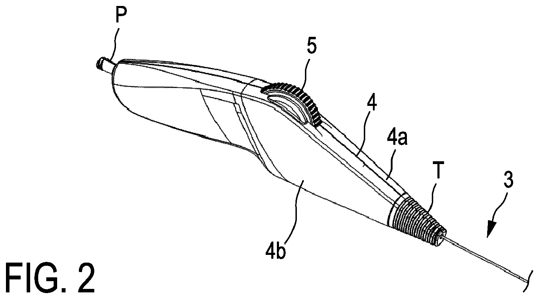

In the present case, the housing 4 is manufactured from a dimensionally stable synthetic material. As it is further apparent with reference to Figs. 1 and 2, the housing 4 provides a handle which may be grasped by a hand, such that the thumb of the respective hand is placed in the region of an upper side of the housing 4 for rotating actuation of the thumbwheel 5.

The thumbwheel 5 is mounted in the housing 4 to be rotatable about a rotation axis D (Fig. 3) and protrudes, in the radial direction of the thumbwheel 5, from a recess of the housing 4 not described in more detail. As a result, the thumbwheel 5 is manually operable in the above described manner. The rotation axis D is perpendicular to the proximal displacement direction of the outer sheath 9 and - during conventional handling - in approximately horizontal orientation.

The winding spool 6 is orientated coaxially to the thumbwheel 5 and connected to the thumbwheel in a torque-transmitting manner. As a result, the winding spool 6 is rotatable together with the thumbwheel 5 about the rotation axis D. In a not shown embodiment, the winding spool 6 can be provided integrally with the thumbwheel 5. In the embodiment shown, the winding spool 6 is disposed completely inside the housing 4.

The catheter arrangement 3 extends in the region of the handling device 2 between a distal end and a proximal end of the housing 4 within the latter. The inner shaft 7 includes a lumen, not apparent in more detail, which extends between the proximal end 8 and the distal end 14. In the region of the proximal end 8, the lumen leads into a Luer port P which is fixed to the proximal end of the housing 4 in a generally well-known manner. On its distal end 14, the inner shaft 7 includes a catheter tip. The inner shaft 7 has a flexible design. The outer sheath 9 is manufactured from a braided material, in the embodiment shown, and connected in the region of its proximal end 12 (Fig. 4) to the flexible pull member 11 for transfer of pulling force, in a generally well-known manner. In the embodiment shown, the first stent 10 is a self-expanding stent.

Furthermore, in the present case, the catheter arrangement 3 includes a plurality of stents 10, 10', 10". In that context, in addition to the stent 10 which may also be referred to a first stent 10, a second stent 10' and a third stent 10" are provided, which stents are held in the radial direction between the inner shaft 7 and the outer sheath 9, in a manner corresponding to the first stent 10. The stents 10, 10', 10" are spaced from each other in the axial direction.

Further, the handling device includes a guiding tip T which is disposed on a distal end of the housing 4 and is held between the housing halves 4a, 4b. The catheter arrangement 3 extends, in the region of the distal end of the housing 4, through the guiding tip T in the axial direction.

Furthermore, the handling device 2 includes a locking and unlocking mechanism L which is intended for locking and unlocking the rotational mobility of the thumbwheel 5, and thus also the winding spool 6. However, the locking and unlocking mechanism L is not mandatory. Therefore, a more detailed description thereof is omitted for reasons of brevity.

## Claims

1. Stent delivery system (1), comprising:
- a handling device (2) having a housing (4), a thumbwheel (5) mounted in the housing (4) to be rotatable, and a winding spool (6) rotating together with the thumbwheel (5);
- a catheter arrangement (3) having an inner shaft (7), with a proximal end (8) of the inner shaft (7) fixed on the housing (4), an outer sheath (9) disposed coaxially to the inner shaft (7), and having at least one stent (10) which is received radially between the inner shaft (7) and the outer sheath (9);
- a flexible pull member (11) which, with one end, engages on a proximal end (12) of the outer sheath (9) and, with the other end, is held on the winding spool (6) to be windable;
- wherein, for release of at least the one stent (10), the outer sheath (9) is displaceable relative to the inner shaft (7) in the proximal direction by means of winding the flexible pull member (11) on the winding spool (6);
- **characterized in that** a deflecting and tensioning device (15) is provided spaced from the winding spool (6) in the proximal direction, which deflecting and tensioning device (15) is in operative connection to the flexible pull member (11), and is used to deflect the flexible pull member (11) in a direction towards the winding spool (6) and to tension the flexible pull member (11).

2. Stent delivery system (1) according to claim 1, **characterized in that** the deflecting and tensioning device (15) is disposed completely inside the housing (4).

3. Stent delivery system (1) according to claim 1 or 2, **characterized in that** the deflecting and tensioning device (15) includes a base body (16) fixed in the housing (4) and a tensioning element (17) acting on the flexible pull member (11), which tensioning element (17) is mounted on the base body (16) to be movable relative to the base body (16) between different tensioning positions, wherein the flexible pull member (11) in the different tensioning positions is tensioned by means of the tensioning element (17) to a different extent.

4. Stent delivery system (1) according to claim 3, **characterized in that** the tensioning element (17) is mounted on the base body (16) to be pivotable about a pivoting axis (S).

5. Stent delivery system (1) according to claim 3 or 4, **characterized in that** the tensioning element (17) includes a tensioning section (23) which acts radially on the flexible pull member (11), whereby the flexible pull member (11) is tensioned.

6. Stent delivery system (1) according to any of the claims 3 to 5, **characterized in that** the tensioning element (17) includes a deflecting section (24) on which the flexible pull member (11) is deflected in sliding from an extension in the proximal direction towards the direction of the winding spool (6).

7. Stent delivery system (1) according to any of the claims 3 to 6, **characterized in that** the base body (16) includes a passage (20) extending between a distal end (21) and a proximal end (22) of the base body (16), wherein the inner shaft (7) and the flexible pull member (11) extend through the passage (20).

8. Stent delivery system (1) according to any of the claims 3 to 7, **characterized in that** the deflecting and tensioning device (15) includes a ratchet mechanism (27, 28) provided between the base body (16) and the tensioning element (17), which ratchet mechanism (27, 28) allows movement of the tensioning element (17) between the different tensioning positions in a first direction (R1) to increase the tension of the flexible pull member (11), and counteracts movement of the tensioning element (17) in an opposite second direction (R2).

9. Stent delivery system (1) according to claim 8, **characterized in that** the ratchet mechanism (27, 28) includes a first ratchet surface (27) provided on the base body (16) and a second ratchet surface (28) provided on the tensioning element (17), which second ratchet surface (28) cooperates to register with the first ratchet surface (27) along the first direction (R1), and which is locked in a form-fitting manner against the first ratchet surface (27) along the second direction (R2).

10. Handling device (2) for a stent delivery system (1) according to any of the preceding claims, having a housing (4), a thumbwheel (5) mounted in the housing (4) to be rotatable, and a winding spool (6) rotating together with the thumbwheel (5), wherein a deflecting and tensioning device (15) is provided spaced from the winding spool (6) in a proximal direction, which deflecting and tensioning device (15) is configured for operative connection to a flexible pull member (11) of a catheter arrangement (3) of the stent delivery system (1).
